(19) Europäisches Patentamt
European Patent Office
Office européen des brevets

(11) Publication number: **0 123 541**
**B1**

# (12) EUROPEAN PATENT SPECIFICATION

(45) Date of publication of patent specification: **22.07.87**

(21) Application number: **84302718.6**

(22) Date of filing: **19.04.84**

(51) Int. Cl.[4]: **C 07 C 59/90,** C 07 C 51/347, C 07 C 149/40, C 07 C 147/107, C 07 D 317/14, C 07 D 233/78, C 07 D 207/44, C 07 D 303/02, A 61 K 31/19

## (54) Leukotriene antagonists.

(30) Priority: **21.04.83 US 487434**

(43) Date of publication of application: **31.10.84 Bulletin 84/44**

(45) Publication of the grant of the patent: **22.07.87 Bulletin 87/30**

(84) Designated Contracting States: **CH DE FR GB IT LI NL**

(56) References cited:
**EP-A-0 028 063**
**EP-A-0 104 885**
**EP-A-0 106 565**

(73) Proprietor: **MERCK FROSST CANADA INC.**
**16711 Trans-Canada Highway**
**Kirkland Quebec (CA)**

(72) Inventor: **Belanger, Patrice**
**419 Promenade d'Avignon**
**Dollard des Ormeaux Quebec (CA)**
Inventor: **Guindon, Yvan**
**409 Place Closse**
**Ile Bizard Quebec (CA)**
Inventor: **Fortin, Rejean**
**4820 Leger Blvd.**
**Montreal Quebec (CA)**
Inventor: **Yaokim, Christiane**
**5770 Cote St. Luc Road Apt. 12**
**Montreal H3X 2J1 Quebec (CA)**
Inventor: **Rokach, Joshua**
**416 Canterbury Square**
**Chomedy Laval Quebec (CA)**

(74) Representative: **Crampton, Keith John Allen et al**
**D YOUNG & CO 10 Staple Inn**
**London WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 123 541 B1

Courier Press, Leamington Spa, England.

**Description**

The present invention is concerned with compounds having activity as leukotriene antagonists, methods for their preparation, intermediate compounds useful in their preparation and methods for using these compounds.

Although the chemical identity of leukotrienes was not discovered until 1979, their history actually began in Australia in 1938 when researchers discovered slow reacting substances (SRS) which caused slow contractions of smooth muscle. When their chemically identity was learned, SRS was found to be a mixture of three previously unknown substances which are related chemically to the prostaglandins and thromboxanes. They were named leukotrienes because they are made by leukocytes and have three conjugated double bonds. Leukotrienes have major effects on the smaller peripheral airways of the lungs and on the larger central passages which include the trachea and the bronchi. In the presence of an allergy trigger, like pollen or dust, leukotrienes are manufactured from fatty substances trapped in the membrane of a triggered cell. A series of reactions within the cell generates a set of different leukotrienes which are transported through the cell membrane into the blood. Then they bring about a constriction of the air passages leading to breathlessness. In addition, the leukotrienes have been implicated, as mediators of numerous other disease states, including inflammation, skin diseases and allergic reactions, among others. See, for example: D. M. Bailey *et al.*, *Ann. Rpts. Med. Chem.* *17* 203 (1982).

Several classes of compounds are known to be leukotriene antagonists, see e.g. United Kingdom Patent Specification No. GB—A—2,058,785 and corresponding European Patent Specification No. EP—A—0,028,063, which disclose phenoxyalkoxypnenyl compounds that are useful as antagonists of the slow-reacting substance of anaphylaxis. These compounds have the formula

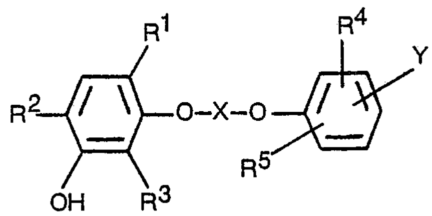

in which the values of the variables include the following:

Y may be —A or —ZA where A is a carboxylic acid group or a 5-1H-tetrazolyl ring and Z may be $C_{1-4}$ alkylene; X may be a saturated $C_{1-10}$ carbon chain that is optionally substituted by a hydroxy group;

$R^1$ may be hydrogen;

$R^2$ is $—COR^6$ where $R^6$ may be $C_{1-6}$ alkyl;

$R^3$ may be $C_{1-6}$ alkyl; and

$R^4$ and $R^5$ may be hydrogen or halogen.

European Patent Specification EP—A—0,104,885 and EP—A—0,106,565 disclose *inter alia* leukotriene antagonists of the formulae

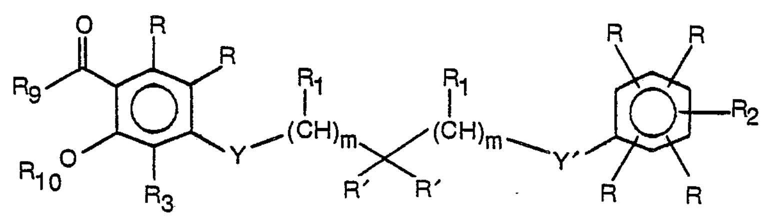

In these formulae, among other values of the variables, R′, $R_1$ and $R_{10}$ can be hydrogen, $R_9$ can be $C_{1-6}$ alkyl, each R can be hydrogen, halogen, hydroxy or $C_{1-6}$ alkyl, $R_3$ can be $C_{1-6}$ alkyl, $R_2$ can be acyl in EP—A—0,106,565 and α-hydroxyalkyl in EP—A—0,104,885, *m* can be from 0 to 6, and Y and Y′ can be O, but neither R nor $R^2$ can be carboxymethyl or an ester thereof.

European Patent Specification No. EP—A—0,056,172 discloses phenoxy and thiophenoxy compounds that are useful as antagonists of the slow-reacting substance of anaphylaxis; European Patent Specification No. EP—A—0,061,800 discloses anti-SRS-A bicyclic carboxylic acid derivatives.

The present invention provides compounds of the formula:

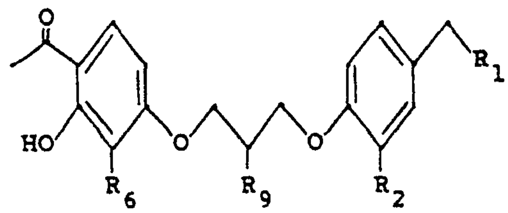

I

in which

$R_1$ is a radical of formula $COOR_3$, where $R_3$ is hydrogen or an alkyl or cycloalkyl group containing not more than six carbon atoms; $CH_2OH$; CHO; $CH_2NHSO_2R_4$, where $R_4$ is OH, $C_{1-6}$ alkyl, $C_{1-6}$ alkoxy, phenyl, phenyl substituted by $C_{1-3}$ alkyl or $C_{1-3}$ alkoxy, halogen, hydroxy, haloalkyl, COOH, CN, formyl, $C_{1-6}$ acyl or $C_{1-4}$ perfluoroalkyl; a tetrazolyl radical; a monocyclic or bicyclic heterocyclic ring containing an acidic hydroxyl group; or a radical of formula:

$$-COO(-CH_2)_s-\underset{R_{10}}{\overset{R_{10}}{\overset{|}{\underset{|}{C}}}}(CH_2)_s-R_7$$

where each s, independently of the other, is 0, 1, 2 or 3, each $R_{10}$, independently of the other, is H or $C_{1-4}$ alkyl, and $R_7$ is a radical $X—R_8$ where X is O, S, or NH and $R_8$ contains up to 21 carbon atoms and is (1) a hydrocarbon radical or (2) an acyl radical derived from an organic acyclic or monocyclic carboxylic acid containing not more than 1 heteroatom that is N, O or S, in the ring;

$R_6$ is H or $C_{1-4}$ alkyl;

$R_2$ is a fluorine or bromine atom; and

$R_9$ is a hydrogen atom or hydroxyl group; and compounds that are pharmaceutically acceptable salts or acid-addition salts thereof.

In the foregoing defnitions in respect of the compounds of the present invention, halogen means F, Cl, Br or I and all radicals containing sufficient carbon atoms may be straight or branched. When $R_9$ is OH, this invention also embodies the racemic compounds and the corresponding individuals (R) and (S) isomers.

Preferably $R_1$ is $COOR_3$ and $R_6$ is $C_{1-4}$ alkyl.

The compounds of the present invention may be prepared by reacting a compound of formula II or III:

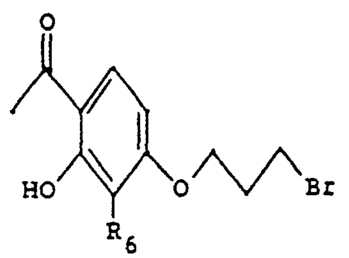

II

or

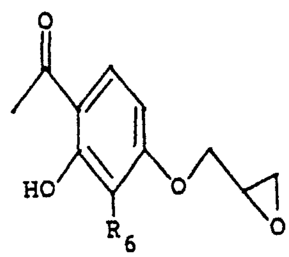

III

with a compound of formula IV

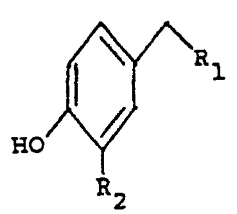

IV

The reaction takes place by refluxing a mixture of the compounds of formulae IV and II or III in an inert solvent such as, for example, methylethylketone (MEK), acetone, tetrahydrofuran (THF), triglyme or dichloromethane in the presence of a base. The reflux temperature is preferably in the range 40 to 130°C. The base may be an alkali metal carbonate, for example, $Li_2CO_3$, $Na_2CO_3$, $Cs_2CO_3$ or $K_2CO_3$.

An alternative procedure involves reacting a compound of formula V or VI prepared by reacting a compound of formula IV with a 1,3-dibromopane or an epihalohydrin under the same conditions as described above, with a compound of formula VII, under the same conditions as above.

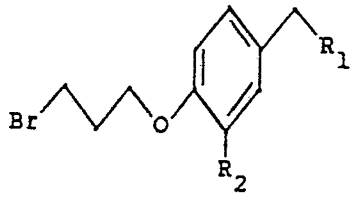

V

or

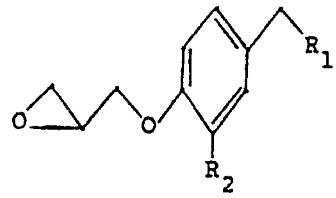

VI

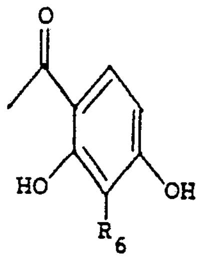

VII

Treatment of the compound of formula I ($R_1$ = COOH) with borane in tetrahydrofuran under known conditions (0°, inert atmosphere) gives the corresponding alcohol, $R_1 = CH_2OH$. Alternatively, borane reduction of a compound of formula IV, V or VI under the same conditions gives the corresponding alcohol (IV, V, VI, $R_1 = CH_2OH$) which are then converted following procedures described above to a compound of formula I ($R_1 = CH_2OH$).

Oxidation of the alcohol of formula I ($R_1 = CH_2OH$) by chromium trioxide in pyridine gives the corresponding aldehyde ($R_1$ = RCHO).

Reacting a compound of formula I ($R_1$ = COOH) with the appropriate alkanol under acid catalysis yields the corresponding ester of formula I ($R_3$ = alkyl).

Reacting the ester of formula I ($R_3$ = alkyl) with ammonia, hydroxyl amine or alkyl- or dialkylamines in a suitable solvent such as THF yields the corresponding compound of formula I ($R_5$ = H, OH or alkyl).

Dehydrating the amide of a compound of formula I ($R_5$ = H) by treatment with phosphorus pentoxide yields the corresponding nitrile of formula I ($R_1$ = CN).

Reacting the foregoing nitrile with sodium azide and $NH_4Cl$ in DMF gives the compound of formula I ($R_1$ = tetrazole).

Treating the compound of formula I ($R_1 = CH_2OH$) with triphenylphosphine and $CBr_4$ in an inert solvent such as diethylether gives the corresponding compound of formula I ($R_1 = CH_2Br$) which in turn by treatment with excess $NH_3$ gives the compound of formula I ($R_1 = CH_2NH_2$).

Treating the foregoing amine with a compound of $R_4SO_2Cl$ in a basic solvent such as pyridine gives the compound of formula I ($R_1 = R_4SO_2NHCH_2$).

Pro-drug esters wherein $R_1$ is a monocyclic or bicyclic heterocylic ring containing an acidic hydroxyl group are obtained by reacting the foregoing heterocycle with a compound of formula I ($R_1$ = COOH) in the presence of dicyclohexylcarbodiimide and hydroxybenzotriazole in an inert solvent such as DMF.

Pro-drug esters wherein $R_1$ is

$$-COO(-CH_2)_s-\overset{\displaystyle R_6}{\underset{\displaystyle R_6}{\overset{|}{\underset{|}{C}}}}-(CH_2)_s-R_7$$

are obtained by reacting the sodium salt of I ($R_1$ = COONa) with

$$Br-(CH_2)_s-\overset{\displaystyle R_6}{\underset{\displaystyle R_6}{\overset{|}{\underset{|}{C}}}}-(CH_2)_s-R_7$$

in an inert solvent such as DMF.

Pharmaceutically acceptable salts of the compound described herein are included within the scope of the present invention. Such salts may be prepared from pharmaceutically acceptable non-toxic bases including inorganic bases and organic bases. Salts derived from inorganic bases include sodium, potassium, lithium, ammonium, calcium, magnesium, ferrous, zinc, copper, manganous, aluminum, ferric,

manganic salts and the like. Particularly preferred are the potassium, sodium, calcium, and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, tri-methylamine, diethanolamine, diethylamine, triethylamine, tripropylamine, ethanolamine, 2-dimethylaminoethanol, 2-diethylaminoethanol, tomethamine, lysine, arginine, histidine, caffeine, procaine, hydrabamine, choline, imidazole, betaine, ethylenediamine, glucosamine, methylglucamine, theobromine, purines, piperazine, N,N'-dibenzylethylenediamine, piperidine, N-ethyl-piperidine, morpholine, N-ethylmorpholine, polyamine resins and the like.

Biological Activity

The compounds of formula I and pharmaceutically acceptable derivatives are therapeutically useful, since they antagonize *in vitro* and *in vivo* actions of leukotriene $D_4$ (a component of SRS). Thus these products will be useful in the treatment of pathological states in which leukotriene $D_4$ and related products are involved. This includes asthma and other allergic disease states, including allergic rhinitis; hypersensitivity (inflammation) reactions in skin, e.g. psoriasis and atopic eczema, lung and gastrointestinal tract; vascular and cardiovascular disorders such as angina; and other disorders such as chronic bronchitis where leukotriene actions are detrimental.

The antagonist activity of the compounds of the present invention was determined by measuring their ability to inhibit leukotriene $D_4$ induced bronochoconstriction in anaesthetized guinea pigs (modified Konzett-Rossler). When administrated directly into the duodenum (oral activity) they are active at 2.5—5 mg/kg. Oral activity is defined as the dose producing 50% inhibition when given 10 minutes before $LTD_4$. Percent inhibition was determined at 10 minutes and also at 30 and 50 minutes after antagonist administration. The time interval between $LTD_4$ administration was kept constant at 20 minutes in all experiments.

Table I is a comparison between three known leukotriene antagonists (entries 1, 2 and 3, see: G.B. 2,058,785, *supra*) and several compounds of the present invention (4—9). The median effective dose ($ED_{50}$ in mg/kg) required for the compounds of the present invention is either equal to or less than that of the prior art compounds. The percentage of bronchoconstriction inhibition of the compounds of the present invention, combined with the effective dose, demonstrates the superiority of the compounds of the present invention over the analogous prior art compounds.

TABLE I
Comparison of compounds of the present invention with known compounds in Inhibiting Bronchoconstriction

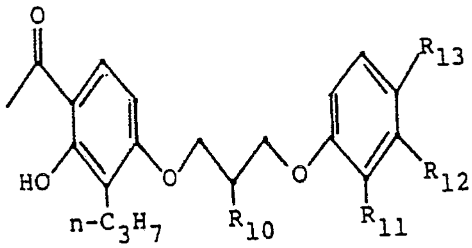

| Entry | $R_{10}$ | $R_{11}$ | $R_{12}$ | $R_{13}$ | $ED_{50}$ | % Inhibition After 30 Min. |
|---|---|---|---|---|---|---|
| 1 | H | H | H | COOH | 10 | 59 |
| 2 | H | H | $(CH_2)_2COOH$ | H | 10 | 32 |
| 3 | H | H | H | $CH = CHCO_2H$ | 20 | 56 |
| 4* | H | F | H | $CH_2COOH$ | 10 | 100 |
| 5* | OH | F | H | $CH_2COOH$ | 2.5 | 50 |
| 6* | OH | Br | H | $CH_2COOH$ | 2.5 | 35 |
| 7* | OH | Cl | H | $CH_2COOH$ | 10 | 63 |
| 8* | H | Cl | H | $CH_2COOH$ | 5 | 60 |
| 9* | H | Br | H | $CH_2COOH$ | 10 | 87 |

* Compounds of the present invention.

The magnitude of a prophylactic or therapeutic dose of a compound of formula I will, of course, vary with the nature of the severity of the condition to be treated and with the particular compound of formula I and its route of administration. In general, the daily dose range lies within the range of from about 0.2 mg to about 100 mg per kg body weight of a mammal.

The pharmaceutical compositions of the present invention comprises a compound of formula I or a pharmaceutically acceptable salt thereof as an active ingredient, and may also contain a pharmaceutically acceptable carrier and optionally other therapeutic ingredients.

The term "pharmaceutically acceptable salts" refers to salts prepared from pharmaceutically acceptable non-toxic bases including inorganic bases and organic bases. Salts derived form inorganic bases include sodium, potassium, lithium, ammonium, calcium, magnesium, ferrous, zinc, copper, manganous, aluminum, ferric, manganic salts and the like. Particularly preferred are the ammonium, potassium, sodium, calcium and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, tromethamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like.

The compositions include compositions suitable for oral, rectal, ophthalmic, pulmonary, nasal, dermal, topical or parenteral (including subcutaneous, intramuscular and intra-venous) administration, although the most suitable route in any given case will depend on the nature and severity of the conditions being treated and on the nature of the active ingredient. They may be conveniently presented in unit dosage form and prepared by any of the methods well-known in the art of pharmacy.

For use where a composition for intravenous administration is employed, a suitable dosage range is from about 0.2 to about 20 mg (preferably from about 1 to about 10 mg) of a compound of formula I per kg of body weight per day and in the case where an oral composition is employed a suitable dosage range is

e.g. from about 1 to about 100 mg of a compound of formula I per kg of body weight per day, preferably from about 5 to about 40 mg/kg.

For administration by inhalation, the compounds of the present invention are conveniently delivered in the form of an aerosol spray presentation from pressurized packs or a nebuliser. The preferred composition for inhalation is a powder which may be formulated as a cartridge from which the powder composition may be inhaled with the aid of a suitable device. In the case of a pressurized aerosol, the dosage unit may be determined by providing a valve to deliver a metered amount.

Pharmaceutical compositions of the present invention suitable for oral administration and by inhalation in the case of asthma therapy may be presented as discrete units such as capsules, cachets, or tablets each containing a predetermined amount of the active ingredients, as a powder or granules, or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredients with liquid carriers or finely divided solid carriers or both, and then, if necessary, shaping the product into the desired presentation. For example, a tablet may be prepared by compression or moulding, optionally with one or more accessory ingredients. Compressed tablets may be prepared by compressing in a suitable machine, the active ingredient in a free-flowing form such as powder or granules, optionally mixed with a binder, lubricant, inert diluent, lubricating, surface active or dispersing agent. Moulded tablets may be made by moulding in a suitable machine, a mixture of the powdered compound moistened with an inert liquid diluent. Desirably, each tablet contains from about 25 mg to about 500 mg of the active ingredient and each cachet or capsule contains from about 25 to about 500 mg of the active ingredient.

In addition to the compounds of Formula I, the pharmaceutical compositions of the present invention can also contain other active ingredients, such as cyclooxygenase inhibitors, non-steroidal anti-inflammatory drugs (NSAIDs), peripheral analgesic agents such as zomepirac diflunisal and the like. The weight ratio of the compound of the Formula I to the second active ingredient may be varied and will depend upon the effective dose of each ingredient. Generally, an effective dose of each will be used. Thus, for example, when a compound of the Formula I is combined with an NSAID the weight ratio of the compound of the Formula I to the NSAID will generally range from about 200:1 to about 1:200. Combinations of a compound of the Formula I and other active ingredients will generally also be within the aforementioned range, but in each case, an effective dose of each active ingredient should be used.

NSAIDs can be characterized into five groups:

(1) the propionic acid derivatives;
(2) the acetic acid derivatives;
(3) the fenamic acid derivatives;
(4) the biphenylcarboxylic acid derivatives; and
(5) the oxicams

or a pharmaceutically acceptable salt thereof.

The propionic acid derivatives which may be used comprise: ibuprofen, ibuprufen aluminum, indoprofen, ketoprofen, naproxen, benoxaprofen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen and bucloxic acid. Structurally related propionic acid derivatives having smaller analgesic and anti-inflammatory properties are also intended to be included in this group.

Thus, "propionic acid derivatives" as defined herein are non-narcotic analgesics/non-steroidal anti-inflammatory drugs having a free $—CH(CH_3)COOH$ or $—CH_2CH_2COOH$ group (which optionally can be in the form of a pharmaceutically acceptable salt group, e.g., $—CH(CH_3)COO^-Na^+$ or $—CH_2CH_2COO^-Na^+$), typically attached directly or via a carbonyl function to a ring system, preferably to an aromatic ring system.

The acetic acid derivatives which may be used comprise: indomethacin, which is a preferred NSAID, sulindac, tolmetin, zomepirac, diclofenac, fenclofenac, alclofenac, ibufenac, isoxepac, furofenac, tiopinac, zidometacin, acemetacin, fentiazac, clidanac, oxpinac, and fenclozic acid. Structually related acetic acid derivatives having similar analgesic and antiinflammatory properties are also intended to be encompassed by this group.

Thus, "acetic acid derivatives" as defined herein are non-narcotic analgesics/non-steroidal anti-inflammatory drugs having a free $—CH_2COOH$ group (which optionally can be in the form of a pharmaceutically acceptable salt group, e.g. $—CH_2COO^-Na^+$), typically attached directly to a ring system, preferably to an aromatic or heteroaromatic ring system.

The fenamic acid derivatives which may be used comprise: mefenamic acid, meclofenamic acid, flufenamic acid, niflumic acid and tolfenamic acid. Structurally related fenamic acid derivatives having similar analgesic and anti-inflammatory properties are also intended to be encompassed by this group.

Thus, "fenamic acid derivatives" as defined herein are non-narcotic analgesics/non-steroidal anti-inflammatory drugs which contain the basic structure:

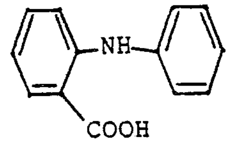

which can bear a variety of substituents and in which the free —COOH group can be in the form of a pharmaceutically acceptable salt group, e.g., —$COO^-Na^+$.

The biphenylcarboxylic acid derivatives which can be used comprise: diflunisal and flufenisal. Structurally related biphenylcarboxylic acid derivatives having similar analgesic and anti-inflammatory properties are also intended to be encompassed by this group.

Thus, "biphenylcarboxylic acid derivatives" as defined herein are non-narcotic analgesics/non-steroidal anti-inflammatory drugs which contain the basic structure:

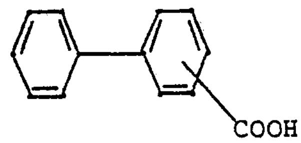

which can bear a variety of substituents and in which the free —COOH group can be in the form of a pharmaceutically acceptable salt group, e.g., —$COO^-Na^+$.

The oxicams which can be used in the present invention comprise: piroxicam, sudoxicam, isoxicam and 4-hydroxyl-1,2-benzothiazine 1,1-dioxide 4-(N-phenyl)-carboxamide. Structurally related oxicams having similar analgesic and anti-inflammatory properties are also intended to be encompassed by this group.

Thus, "oxicams" as defined herein are non-narcotic analgesics/non-steroidal anti-inflammatory drugs which have the general formula:

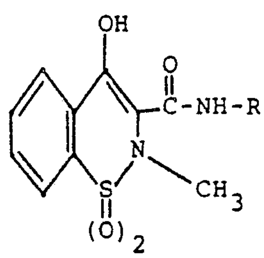

wherein R is an aryl or heteroaryl ring system.

The following NSAIDs may also be used: acemetacin, alminoprofen, amfenac sodium, aminoprofen, anitrazafen, antrafenine, auranofin, bendazac lysinate, benzydamine, beprozin, broperamole, bufezolac, carprofen, cinmetacin, ciproquazone, clidanac, cloximate, dazidamine, deboxamet, delmetacin, detomidine, dexindoprofen, diacerein, di-fisalamine, difenpyramide, emorfazone, enfenamic acid, enolicam, epirizole, etersalate, etodolac, etofenamate, fanetizole mesylate, fenclofenac, fenclorac, fendosal, fenflumizole, fentiazac, feprazone, floctafenine, flunixin, flunoxaprofen, fluproquazone, fopirtoline, fosfosal, furcloprofen, furofenac, glucametacin, guaimesal, ibuproxam, isofezolac, isonixim, isoprofen, isoxepac, isoxicam, lefetamine HCl, leflunomide, lofemizole, lonazolac calcium, lotifazole, loxoprofen, lysin clonixinate, meclofenamate sodium, meseclazone, miroprofen, nabumetone, nictindole, nimesulide, orpanoxin, oxametacin, oxapadol, oxaprozin, perisoxal citrate, pimeprofen, pimetacin, piproxen, pirazolac, pirfenidone, pirprofen, pranoprofen, proglumetacin maleate, proquazone, pyridoxiprofen, sudoxicam, suprofen, talmetacin, talniflumate, tenoxicam, thiazolinobutazone, thielavin B, tiaprofenic acid, tiaramide HCl, tiflamizole, timegadine, tioxaprofen, tolfenamic acid, tolpadol, tryptamid, ufenamate, and zidometacin.

The following NSAIDs, designated by company code number and systematic chemical name, may also be used:

| | |
|---|---|
| 480156S: | 2-[4-(2-thiazolyloxy)-phenyl]-propionic acid |
| AA861: | 2,3,5-trimethyl-6-(12-hydroxy-5,10-dodecadiynyl-1,4-benzoquinone |
| AD1590: | 2-(8-methyl-10,11-dihydro-11-oxodibenz[b,f]oxepin-2-yl)propionic acid |
| AFP802: | 3,4,5-trimethoxybenzoyl-ibuprofen |
| AFP860: | guaiphenesin ibuprofanate |

| | |
|---|---|
| AI77B: | [6-[[1-(3,4-dihydro-8-hydroxy-1-oxo-1H-2-benzopyran-3-yl)-3-methylbutyl]amino]-5-dihydroxy-6-oxo-3-hexanamido]ammonium chloride |
| AP504: | chlorhexidine dinaproxenate |
| AU8001: | 4'-acetamidophenyl-2-(5'-*p*-toluyl-1'-methyl-pyrrole)-acetate |
| BPPC: | (±)-5-benzoyl-2,3-dihydro-1H-pyrrolizine-1-carboxylic acid trometamol salt |
| BW540C: | 3-methylamino-1-[3-(trifluoromethyl)-phenyl]-2-pyrazoline |
| CHINOIN 127: | a rimazolium analogue |
| CN100: | under development by Nippon Chemiphar |
| EB382: | α-Methyl-4-[(2-methyl-2-propenyl)amino]benzeneacetic acid |
| EL508: | (±-α-[[(2-hydroxy-1,1-dimethylethyl)amino]methyl]benzyl alcohol |
| F1044: | 5-[5-(4-chlorophenyl)-2-furanyl]-dihydro-2(3H)-furanone |
| GV3658: | diflunisal lysine salt |
| ITF182: | imidazole 2-hydroxybenzoate |
| KCNTEI6090: | methyl 5,6-E-5,6-methanoeicosa-7E,9E,11Z,14E-eicosatetranoate |
| KME4: | 3,5-di-*t*-butyl-4-hydrobenzylidene-γ-butyrolactone |
| LA2851: | 2,4-diamino-7-methyl-pyrazolo(1,5-a)-1,3,5-triazine |
| MR714: | 2-(2',4'-difluoro-4-biphenyl)oxypropionic acid |
| MR897: | 3-methyl-3-(4-acetyl-aminophenoxy)-2,4-dioxobenzocyclo-1-esanone |
| MY309: | 4-(p-chlorophenyl)-1-(p-fluorophenyl)pyrazole-3-acetic acid |
| ONO3144: | 2-aminomethyl-4-*t*-butyl-6-propionylphenol hydrochloride |
| PR823: | glycolic acid[o-(2,6-dichloroanilino)phenyl]acetate(ester) |
| PV102: | (±)-1-(p-chlorobenzyl)-5-methoxy-2-methylindole-3-acetic acid ester with 3-hydroxy phthalide |
| PV108: | phthalidyl ester of diclofenac |
| R830: | 2,6-di-*t*-butyl-4-(2'-thenoyl)phenol |
| RS2131: | (2-(4-(2-oxocyclohexylidinemethyl)phenyl)propionic acid |
| SCR152: | 2-(4-biphenylyl)-4-hexenoic acid |
| SH440: | 11β,17,21-trihydroxy-6α-methylpregna-1,4-diene-3,20-dione 21-acetate 17-propionate |
| SIR133: | 4-(2-bromo-4,5-dimethoxyphenyl)-octahydro-2H-quinolizin-2-one |
| SPAS510: | N-(2-pyridyl)-2-methyl-4-cinnamoyloxy-2H-1,2-benzothiazine-3-carboxamide 1,1-dioxide |
| SQ27239: | 17α-ethylthio-9α-fluoro-11β-hydroxy-17β-methylthio-androsta-1,4-dien-3-one |
| ST281: | 1,1,3-trimethyl-5-phenylbiuret |
| SY6001: | 1-(4-chlorobenzoyl)-5-methoxy-2-methyl-1H-indole-3-acetic acid butanediol ester |
| TA60: | 2-(4-(3-methyl-2-butenyl)phenyl)propionic acid |
| TVX2706: | 3-ethyl-1-(3-nitrophenyl)-2,4-[1H,3H]-quinazolindione |
| U60257: | (6,9-de-epoxy-6,9-(phenylimino)-$\Delta^{6,8}$-PG11 (Piriprost) |
| UR2301: | 1-benzoyl-5-methoxy-2-methylindole-3-acetic acid |
| WY41770: | (5H-dibenzo[a,d]cyclohepten-5-ylidene)acetic acid |

Finally, NSAIDS which may also be used include the salicylates, specifically aspirin, and the phenylbutazones, and pharmaceutically acceptable salts thereof.

Pharmaceutical compositions comprising the Formula I compounds may also contain inhibitors of the biosynthesis of the leukotrienes such as are disclosed in pending U.S. Patent Applications Nos. 539,342, 459,924, 539,215, 547,161.

The compounds of Formula I may also be used in combination with leukotriene antagonists such as those disclosed in copending U.S. Patent Applications Nos. 520,051 and 520,052, and others such as those disclosed in European Patent Specifications EP—A—0,056,172 and EP—A—0,061,800; and in U.K. Patent Specification GB—A—2,058,785.

Pharmaceutical compositions comprising the Formula I compounds may also contain as the second active ingredients antihistaminic agents such as benadryl, dramamine, histadyl and phenergan. Alternatively, they may include prostaglandin antagonists such as those disclosed in European Patent Specification EP—A—0,011,067 or thromboxane antagonists such as those disclosed in U.S. Patent Specification US—A—4,237,160. They may also contain histidine decarboxyase inhibitors sych as α-fluoromethylhistidine, described in U.S. Patent Specification US—A—4,325,961. The compounds of the Formula I may also be advantageously combined with an $H_1$ or $H_2$-receptor antagonist, such as for instance cimetidine, ranitidine, terfenadine, famotidine, aminothiadiazoles disclosed in European Patent Application 81102976.8 and like compounds, such as those disclosed in U.S Patents US—A—4,283,408; 4,362,736 and 4,394,508; European Patent Specification EP—A—0,400,696 and a pending U.S. Application No. 301,616. The pharmaceutical compositions may also contain a $K^+/H^+$ ATPase inhibitor such as omeprazole, disclosed in U.S. Patent Specification US—A—4,255,431.

The following examples illustrate but do not limit the present invention.

## Example 1

4-(3-(4-Acetyl-3-hydroxy-2-propylphenoxy)-2-hydroxypropoxy)-3-fluorobenzene acetic acid

*Step A:* Preparation of 4-(3-(4-acetyl-3-hydroxypropyl-2-phenoxy)-2-hydroxypropoxy)-3-fluorobenzene acetic acid methyl ester

A mixture of 4-(2,3-epoxypropoxy)-3-propyl-2-hydroxyacetophenone (1.25 g, 5 mmoles) and 3-fluoro-4-hydroxybenzene acetic acid methyl ester (0.92 g, 5 mmoles) and potassium carbonate (1.38 g, 10 mmoles) in methyl ethyl ketone (25 ml) was heated under reflux for 48 hours.

The reaction mixture was filtered, the solid was washed with acetone and the filtrate was evaporated to dryness. The residue was then chromatographed on silica gel to yield, on eluting with 20% EtOAc/hexane, 1.3 g of the title compound.

| | | | |
|---|---|---|---|
| Elemental Analysis, Calc'd: | C, 63.58; | H, 6.26; | F, 4.37; |
| Obtained: | C, 63.51; | H, 6.20; | F, 4.24 |

*Step B:* Preparation of 4-(3-(4-acetyl-3-hydroxy-2-propylproxy)-3-fluorobenzene acetic acid

The ester prepared in Step A, (1.1 g, 2.53 mmoles) dissolved in methanol (25 ml) containing 10N sodium hydroxide (1 ml) was refluxed gently for 15 minutes. The volatiles were removed *in vacuo* and the residue was taken up in water (50 ml). The solution was acidified with 20% citric acid and the mixture was extracted with EtOAc. The extracts were washed with brine, dried ($Na_2SO_4$) and concentrated *in vacuo* to yield 0.9 g of the title compound, m.p. 137—138°C

| | | | |
|---|---|---|---|
| Elemental Analysis, Calc'd: | C, 62.84; | H, 5.99; | F, 4.52; |
| Obtained: | C, 62.73; | H, 5.91; | F, 4.70 |

## Example 2

4-(3-(4-Acetyl-3-hydroxy-2-propylphenoxy)-2-hydroxypropoxy)-3-chlorobenzene acetic acid

*Step A:* Preparation of 4-(3-(4-acetyl-3-hydroxy-2-propylphenoxy)-2-hydroxypropoxy)-3-chlorobenzene acetic acid methyl ester

By following Step A of Example 1, but substituting 3-chloro-4-hydroxy benzene acetic methyl ester for 3-fluoro-4-hydroxy benzene acetic methyl ester, the title compound was obtained as an oil.

| | | | |
|---|---|---|---|
| Elemental Analysis, Calc'd: | C, 61.26; | H, 6.04; | Cl, 7.86; |
| Obtained: | C, 61.06; | H, 6.15; | Cl, 7.93 |

*Step B:* Preparation of 4-(3-(4-acetyl-3-hydroxy-2-propylphenoxy)-2-hydroxypropoxy)-3-chlorobenzene acetic acid

By following Step B of Example 1, but substituting the title compound of Step A of this Example for the title compound of Step A of Example 1, the title compound was obtained, as an oil.

| | | | |
|---|---|---|---|
| Elemental Analysis, Calc'd: | C, 60.48; | H, 5.77; | Cl, 8.12; |
| Obtained: | C, 60.38; | H, 5.70; | Cl, 8.11 |

## Example 3

4-(3-(4-Acetyl-3-hydroxy-2-propylphenoxy)-2-hydroxypropoxy)-3-bromobenzene acetic acid

*Step A:* Preparation of 4-(3-(4-acetyl-3-hydroxy-2-propylphenoxy)-2-hydroxypropoxy)-3-bromobenzene acetic acid methyl ester

By following Step A of Example 1, but substituting 3-bromo-4-hydroxy benzene acetic methyl ester for 3-fluoro-4-hydroxy benzene acetic methyl ester, the title compound was obtained as an oil.

| | | | |
|---|---|---|---|
| Elemental Analysis, Calc'd: | C, 55.77; | H, 5.49; | Br, 16.13; |
| Obtained: | C, 55.61; | H, 5.41; | Br, 16.39 |

*Step B:* Preparation of 4-(3-(4-acetyl-3-hydroxy-2-propylphenoxy)-2-hydroxypropoxy)-3-bromobenzene acetic acid

By following Step B of Example 1, but substituting the title compound of Step A of this Example for the title compound of Step A of Example 1, the title compound was obtained, m.p. 84—84°C.

| | | | |
|---|---|---|---|
| Elemental Analysis, Calc'd: | C, 54.90; | H, 5.24; | Br, 16.60; |
| Obtained: | C, 54.63; | H, 5.01; | Br, 16.93 |

## Example 4

4-(3-(4-Acetyl-3-hydroxy-2-propylphenoxy)-propoxy)-3-fluorobenzene acetic acid

*Step A:* Preparation of 4-(3-(4-acetyl-3-hydroxy-2-propylphenoxy)-propoxy)-3-fluorobenzene acetic acid methyl ester

By following Step A of Example 1, but substituting 4-(3-bromopropoxy)-3-propyl-2-hydroxy acetophenone for 4-(2,3-epoxypropoxy)-3-propyl-2-hydroxy acetophenone, the title compound was obtained as an oil.

| | | | |
|---|---|---|---|
| Elemental Analysis, Calc'd: | C, 65.70; | H, 6.23; | F, 4.52; |
| Obtained: | C, 65.76; | H, 6.06; | F, 4.63 |

*Step B:* Preparation of 4-(3-(4-acetyl-3-hydroxy-2-propylphenoxy)-propoxy)-3-fluorobenzene acetic acid

By following Step B of Example 1, but substituting the title compound of Step A of this Example for the title compound of Step A of Example 1, the title compound was obtained, m.p. 121—123°C.

| | | | |
|---|---|---|---|
| Elemental Analysis, Calc'd: | C, 65.33; | H, 6.23; | F, 4.70; |
| Obtained: | C, 65.49; | H, 6.32; | F, 4.50 |

Example 5

4-(3-(4-Acetyl-3-hydroxy-2-propylphenoxy)-2-propoxy)-3-chlorobenzene acetic

*Step A:* Preparation of 4-(3-(4-acetyl-3-hydroxy-2-propylphenoxy)-2-propoxy)-3-chlorobenzene acetic methyl ester

By following Step A of Example 4, but substituting 3-chloro-4-hydroxy benzene acetic methyl ester for 3-fluoro-4-hydroxy benzene acetic methyl ester, the title compound was obtained as an oil.

| | | | |
|---|---|---|---|
| Elemental Analysis, Calc'd: | C, 63.51; | H, 6.26; | Cl, 8.15; |
| Obtained: | C, 63.78; | H, 6.62; | Cl, 8.37 |

*Step B:* Preparation of 4-(3-(4-acetyl-3-hydroxy-2-propylphenoxy)propoxy)-3-chlorobenzene acetic

By following Step B of Example 1, but substituting the title compound of Step A of this Example for the title compound of Step A of Example 1, the title compound was obtained, m.p. 110—111°C.

| | | | |
|---|---|---|---|
| Elemental Analysis, Calc'd: | C, 62.78; | H, 5.99; | Cl, 8.42; |
| Obtained: | C, 62.60; | H, 6.32; | Cl, 8.37 |

Example 6

4-(3-(4-Acetyl-3-hydroxy-2-propylphenoxy)-propoxy)-3-bromobenzene acetic acid

*Step A:* Preparation of 4-(3-(4-acetyl-3-hydroxy-2-propylphenoxy)-propoxy)-3-bromobenzene acetic acid methyl ester

By following Step A of Example 4, but substituting 3-bromo-4-hydroxy benzene acetic methyl ester for 3-fluoro-4-hydroxy benzene acetic methyl ester, the title compound was obtained, m.p. 91—92°C.

| | | | |
|---|---|---|---|
| Elemental Analysis, Calc'd: | C, 57.63; | H, 5.68; | Br, 16.67; |
| Obtained: | C, 57.35; | H, 5.72; | Br, 16.35 |

*Step B:* Preparation of 4-(3-(4-acetyl-3-hydroxy-2-propylphenoxy)-propoxy)-3-bromobenzene acetic acid

By following Step B of Example 1, but substituting the title compound of Step A of this Example for the title compound of Step A of Example 1, the title compound was obtained, m.p. 111—112°C.

| | | | |
|---|---|---|---|
| Elemental Analysis, Calc'd: | C, 56.78; | H, 5.42; | Br, 17.17; |
| Obtained: | C, 56.82; | H, 5.34; | Br, 17.22 |

Example 7

Following the general procedures described hereinabove, the following compounds are prepared:

4-(3-(4-Acetyl-3-hydroxy-2-propylphenoxy-2-hydroxypropoxy)-3-fluorobenzene acetic acid ethyl ester;

4-(3-(4-Acetyl-3-hydroxy-2-propylphenoxy-2-hydroxypropoxy)-3-fluorobenzenethanol;

4-(3-(4-Acetyl-3-hydroxy-2-propylphenoxy-2-hydroxypropoxy)-3-fluorobenzeneacetaldehyde;

4-(3-(4-Acetyl-3-hydroxy-2-propylphenoxy-2-hydroxypropoxy)-3-fluorobenzenemethyltetrazole;

4-(3-(4-Acetyl-3-hydroxy-2-propylphenoxy-2-hydroxypropoxy)-3-fluorobenzeneethylamine;

N-Methylsulfonyl-4-(3-(4-acetyl-3-hydroxy-2-propylphenoxy-2-hydroxypropoxy)-3-fluorobenzeneethylamine;

4-(3-(4-Acetyl-3-hydroxy-2-propylphenoxy-2-hydroxypropoxy)-3-fluorobenzylnitrile;

N,N-Dimethyl-4-(3-(4-acetyl-3-hydroxy-2-propylphenoxy-2-hydroxypropoxy)-3-fluorobenzeneacetamide;

4-(3-(4-Acetyl-3-hydroxy-2-propylphenoxy-2-hydroxypropoxy)-3-fluorobenzene acetic acid, 4-methoxy-1,2,5-thiadiazol-3-yl-ester.

**Claims**

1. Compounds having the formula:

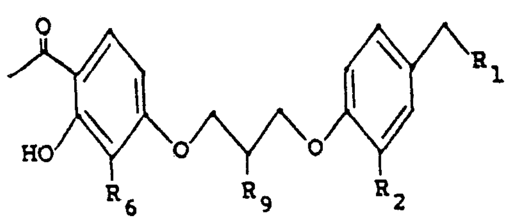

I

in which

$R_1$ is a radical of formula $COOR_3$, where $R_3$ is hydrogen or an alkyl or cycloalkyl group containing not

more than six carbon atoms; $CH_2OH$; CHO; $CH_2NHSO_2R_4$, where $R_4$ is OH, $C_{1-6}$ alkyl, $C_{1-6}$ alkoxy, phenyl, phenyl substituted by $C_{1-3}$ alkyl or $C_{1-3}$ alkoxy, halogen, hydroxy, haloalkyl, COOH, CN, formyl, $C_{1-6}$ acyl or $C_{1-4}$ perfluoroalkyl; a tetrazolyl radical; a monocyclic or bicyclic heterocyclic ring containing an acidic hydroxyl group; or a radical of formula

$$-COO(-CH_2)_s-\underset{R_{10}}{\overset{R_{10}}{C}}(CH_2)_s-R_7$$

where each s, independently of the other, is 0, 1, 2 or 3; $R_7$ is a radical $X-R_8$ where X is O, S or NH and $R_8$ contains up to 21 carbon atoms and is (1) a hydrocarbon radical or (2) an acyl radical derived from an organic acyclic or monocyclic carboxylic acid containing not more than 1 heteroatom that is N, O or S in the ring;
and each $R_{10}$, independently of the other is H or $C_{1-4}$ alkyl;

$R_6$ is H or $C_{1-4}$ alkyl;

$R_2$ is a fluorine or bromine atom; and

$R_9$ is a hydrogen atom or hydroxyl group; and compounds that are pharmaceutically acceptable salts or acid-addition salts thereof.

2. A compound as claimed in Claim 1 in which $R^7$ is $X-R^8$ and X is S.

3. A compound as claimed in Claim 1 in which $R^7$ is $X-R^8$ and X is O.

4. A compound as claimed in Claim 1 in which $R^7$ is $X-R^8$ and X is NH.

5. The following compounds claimed in Claim 1:

4-(3-(4-Acetyl-3-hydroxy-2-propylphenoxy)-2-hydroxypropoxy)-3-fluorobenzene acetic acid;

4-(3-(4-Acetyl-3-hydroxy-2-propylphenoxy)-2-hydroxypropoxy)-3-bromobenzene acetic aicd;

4-(3-(4-Acetyl-3-hydroxy-2-propylphenoxy)-propoxy)-3-fluorobenzene acetic acid;

4-(3-(4-Acetyl-3-hydroxy-2-propylphenoxy)-propoxy)-3-bromobenzene acetic acid;

4-(3-(4-Acetyl-3-hydroxy-2-propylphenoxy-2-hydroxypropoxy)-3-fluorobenzene acetic acid ethyl ester;

4-(3-(4-Acetyl-3-hydroxy-2-propylphenoxy-2-hydroxypropoxy)-3-fluorobenzenethanol;

4-(3-(4-Acetyl-3-hydroxy-2-propylphenoxy-2-hydroxypropoxy)-3-fluorobenzeneacetaldehyde;

4-(3-(4-Acetyl-3-hydroxy-2-propylphenoxy-2-hydroxypropoxy)-3-fluorobenzenemethyltetrazole;

N-Methylsulfonyl-4-(3-(4-acetyl-3-hydroxy-2-propylphenoxy-2-hydroxypropoxy)-3-fluorobenzeneethylamine;

4-(3-(4-Acetyl-3-hydroxy-2-propylphenoxy-2-hydroxypropoxy)-3-fluorobenzylnitrile;

6. A method of preparing a compound having the formula I set forth in Claim 1 in which $R^9$ is hydrogen that comprises reacting a compound of the formula II

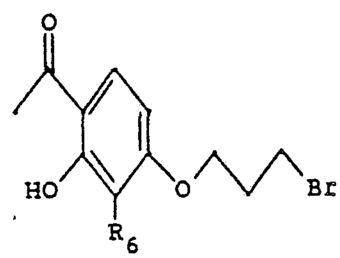

II

with a compound of formula IV

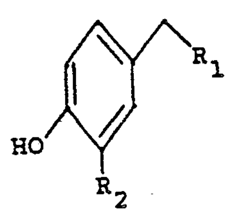

IV

where $R_1$, $R_2$ and $R_6$ are as defined in Claim 1.

7. A method of preparing a compound having the formula I set forth in Claim 1 in which $R^9$ is hydroxy that comprises reacting a compound of the formula III

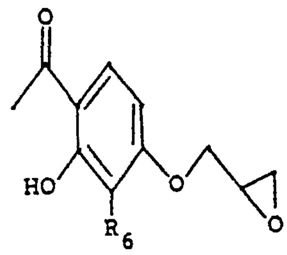

III

with a compound of formula IV

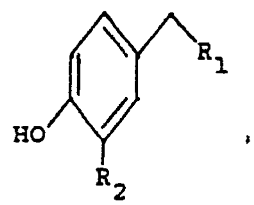

IV

where $R_1$, $R_2$ and $R_6$ are as defined in Claim 1.

8. A method of preparing a compound having the formula I set forth in Claim 1 in which $R^9$ is hydrogen that comprises reacting a compound of the formula V

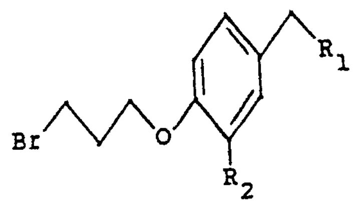

V

with a compound of formula VII

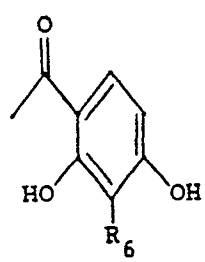

VII

where $R_1$, $R_2$ and $R_6$ are as defined in Claim 1.

9. A method of preparing a compound having the formula I set forth in Claim 1 in which $R^9$ is hydroxy that comprises reacting a compound of the formula VI

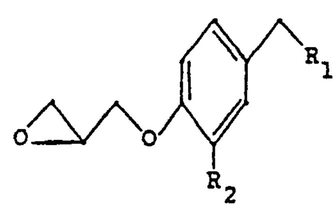

VI

with a compound of formula VII

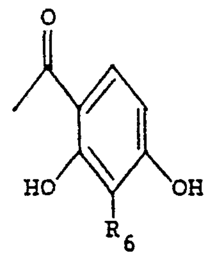

VII

where $R_1$, $R_2$ and $R_6$ are as defined in Claim 1.

10. A composition containing a compound as claimed in Claim 1 and a pharmaceutically acceptable carrier.

11. A pharmaceutical composition comprising a compound as claimed in any one of Claims 1 to 5 and additionally a second active ingredient that is a non-steroidal anti-inflammatory drug, a peripheral analgesic agent, a cyclooxygenase inhibitor, a leukotriene antagonist, a leukotriene inhibitor, an $H_2$-receptor antagonist, an antihistamic agent, a prostaglandin antagonist or a thromboxane antagonist.

12. A composition as claimed in Claim 11 in which the weight ratio of the compound of one of Claims 1 to 5 to the second active ingredient ranges from 200:1 to 1:200.

13. A composition as claimed in Claim 11 or 12 in which the second active ingredient is a non-steroidal anti-inflammatory drug.

14. A composition as claimed in Claim 13 in which the non-steroidal anti-inflammatory drug is indomethacin.

15. A composition as claimed in any one of Claims 10 to 14, for use in antagonizing leukotriene synthesis or mode of action in humans.

16. A composition as claimed in any one of Claims 10 to 14, for use in treating asthma, allergic disorders and inflammation in humans.

**Patentansprüche**

1. Verbindungen mit der Formel

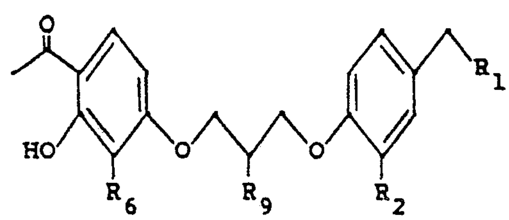

I

in welcher

$R_1$ ein Radikal der Formel $COOR_3$, worin $R_3$ Wasserstoff oder eine nicht mehr als 6 Kohlenstoffatome enthaltende Alkyl- oder Cycloalkylgruppe ist: $CH_2OH$; CHO; $CH_2NHSO_2R_4$, worin $R_4$ OH, $C_{1-6}$-Alkyl, $C_{1-6}$-Alkoxy, Phenyl, durch $C_{1-3}$-Alkyl oder $C_{1-3}$-Alkoxy substituiertes Phenyl, Halogen, Hydroxy, Halogenalkyl, COOH, CN, Formyl, $C_{1-6}$-Acyl oder $C_{1-4}$- Perfluoralkyl ist; ein Tetrazolylradikal; ein monocyclischer oder bicyclischer, eine acide Hydroxylgruppe enthaltender heterocyclischer Ring; oder ein Radikal der Formel

$$-COO(-CH_2)_s-\underset{R_{10}}{\overset{R_{10}}{C}}(CH_2)_s-R_7$$

worin jedes s, unabhängig von dem anderen, 0, 1, 2 oder 3 ist; $R_7$ ein Radikal $X-R_8$ ist, worin X O, S oder NH ist, und $R_8$ bis zu 21 Kohlenstoffatome enthält und (1) ein Kohlenwasserstoffradikal oder (2) ein von einer organischen acyclischen oder monocyclischen Carbonsäure, welche nicht mehr als ein Heteroatom, das N, O oder S ist, im Ring enthält, abgeleitetes Acylradikal ist; und jedes $R_{10}$ unabhängig von dem anderen H oder $C_{1-4}$-Alkyl ist; ist;

$R_6$ H oder $C_{1-4}$-Alkyl ist;

$R_2$ ein Fluor- oder Bromatom ist; und

$R_9$ ein Wasserstoffatom oder eine Hydroxylgruppe ist; sowie Verbindungen, die pharmazeutisch annehmbare Salze oder Säureadditionssalze davon sind.

2. Verbindung nach Anspruch 1, in welcher $R^7$ X—$R^8$ ist und X S ist.
3. Verbindung nach Anspruch 1, in welcher $R^7$ X—$R^8$ ist und X O ist.
4. Verbindung nach Anspruch 1, in welcher $R^7$ X—$R^8$ ist und X NH ist.
5. Die nachstehenden in Anspruch 1 beanspruchten Verbindungen:
4-(3-(4-Acetyl-3-hydroxy-2-propylphenoxy)-2-hydroxypropoxy)-3-fluorbenzolessigsäure;
4-(3-(4-Acetyl-3-hydroxy-2-propylphenoxy)-2-hydroxypropoxy)-3-brombenzolessigsäure;
4-(3-(4-Acetyl-3-hydroxy-2-propylphenoxy)-propoxy)-3-fluorbenzolessigsäure;
4-(3-(4-Acetyl-3-hydroxy-2-propylphenoxy)-propoxy)-3-brombenzolessigsäure;
4-(3-(4-Acetyl-3-hydroxy-2-propylphenoxy-2-hydroxypropoxy)-3-fluorbenzolessigsäureethylester;
4-(3-(4-Acetyl-3-hydroxy-2-propylphenoxy-2-hydroxypropoxy)-3-fluorbenzolethanol;
4-(3-(4-Acetyl-3-hydroxy-2-propylphenoxy-2-hydroxypropoxy)-3-fluorbenzolacetaldehyd;
4-(3-(4-Acetyl-3-hydroxy-2-propylphenoxy-2-hydroxypropoxy)-3-fluorbenzolmethyltetrazol;
N-Methylsulfonyl-4-(3-(4-acetyl-3-hydroxy-2-propylphenoxy-2-hydroxypropoxy)-3-fluorbenzolethylamin;
4-(3-(4-Acetyl-3-hydroxy-2-propylphenoxy-2-hydroxypropoxy)-3-fluorbenzylnitril;

6. Verfahren zur Herstellung einer Verbindung mit der in Anspruch 1 dargestellten Formel I, in welcher $R^9$ Wasserstoff ist, durch Reagieren einer Verbindung der Formel II

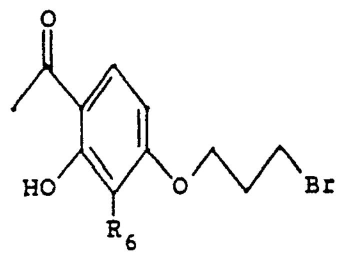

II

mit einer Verbindung der Formel IV

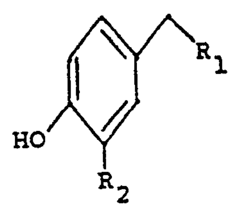

IV

worin $R_1$, $R_2$ und $R_6$ wie in Anspruch 1 definiert sind.

7. Verfahren zur Herstellung einer Verbindung mit der in Anspruch 1 dargestellten Formel I, in welcher $R^9$ Hydroxy ist, durch Reagieren einer Verbindung der Formel III

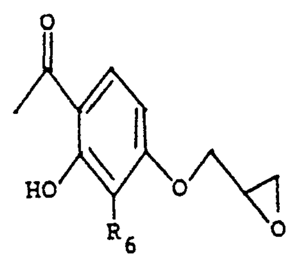

III

mit einer Verbindung der Formel IV

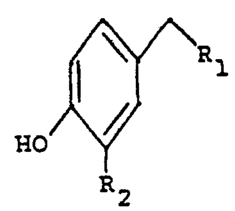

IV

worin $R_1$, $R_2$ und $R_6$ wie in Anspruch 1 definiert sind.

8. Verfahren zur Herstellung einer Verbindung mit der in Anspruch 1 dargestellten Formel I, in welcher $R^9$ Wasserstoff ist, durch Reagieren einer Verbindung der Formel V

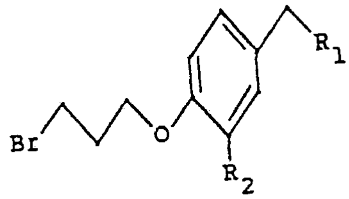

V

mit einer Verbindung der Formel VII

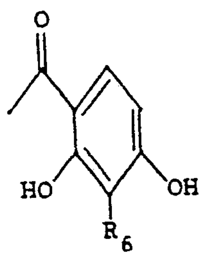

VII

worin $R_1$, $R_2$ und $R_6$ wie in Anspruch 1 definiert sind.

9. Verfahren zur Herstellung eine Verbindung mit der in Anspruch 1 dargestellten Formel I, in welcher $R^9$ Hydroxy ist, durch Reagieren einer Verbindung der Formel VI

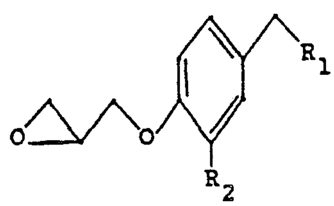

VI

mit einer Verbindung der Formel VII

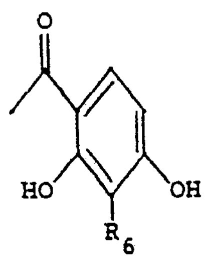

VII

worin $R_1$, $R_2$ und $R_6$ wie in Anspruch 1 definiert sind.

10. Zusammensetzung, enthaltend eine Verbindung nach Anspruch 1 und einen pharmazeutisch annehmbaren Träger

11. Pharmazeutische Zusammensetzung, enthaltend eine Verbindung nach einem der Ansprüche 1 bis 5 und zusätzlich einen zweiten aktiven Bestandteil, welcher ein nicht-steroides entzündungshemmendes Mittel, ein peripheres analgetisches Mittel, ein Cyclooxygenase-Inhibitor, ein Leukotrien-Antagonist, ein Leukotrien-Inhibitor, ein $H_2$-Rezeptor-Antagonist, ein Antihistamin, ein Prostaglandin-Antagonist oder ein Thromboxan-Antagonist ist.

12. Zusammensetzung nach Anspruch 11, in welcher das Gewichtsverhältnis der Verbindung nach einem der Ansprüche 1 bis 5 zu dem zweiten aktiven Bestandteil im Bereich von 200:1 bis 1:200 liegt.

13. Zusammensetzung nach Anspruch 11 oder 12, in welcher der zweite aktive Bestandteil ein nicht-steroides entzündungshemmendes Mittel ist.

14. Zusammensetzung nach Anspruch 13, in welcher das nicht-steroide entzündungshemmende Mittel Indomethacin ist.

15. Zusammensetzung nach einem der Anspruch 10 bis 14 zur Verwendung als Antagonist bei der Leukotrien-Synthese oder -Wirkungsweise im Menschen

16. Zusammensetzung nach einem der Anspruch 10 bis 14 zur Verwendung bei der Behandlung von Asthma, allergischen Störungen und Entzündungen im Menschen.

**Revendications**

1. Composés répondant à la formule:

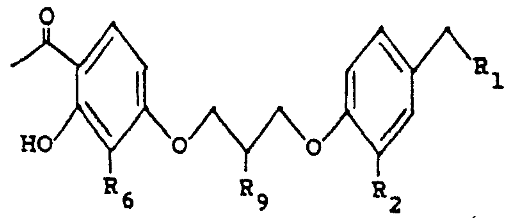

I

dans laquelle

$R_1$ est un radical de formule $COOR_3$ où $R_3$ est un hydrogène ou un groupe alkyle ou cycloalkyle ne contenant pas plus de 6 atomes de carbone; $CH_2OH$; CHO; $CH_2NHSO_2R_4$, où $R_4$ est OH, un alkyle en $C_{1-6}$, un alcoxy en $C_{1-6}$, un phényle, un phényle substitué par un alkyle en $C_{1-3}$ ou un alcoxy en $C_{1-3}$, un halogène, un hydroxy, un halogénoalkyle, COOH, CN, un formyle, un acyle en $C_{1-6}$ ou un perfluoroalkyle en $C_{1-4}$; un radical tétrazolyle; un hétérocycle monocyclique ou bicyclique contenant un groupe hydroxyle acide; ou un radical de formule

$$-COO(-CH_2)_s-\underset{R_{10}}{\overset{R_{10}}{C}}(CH_2)_s-R_7$$

dans laquelle chaque s indépendamment de l'autre est 0, 1, 2 ou 3; $R_7$ est un radical $X-R_8$ où X est O, S ou NH et $R_8$ contient jusqu'à 21 atomes de carbone et est (1) un radical hydrocarboné ou (2) un radical acyle dérivé d'un acide organique carboxylique, acyclique ou monocyclique, ne contenant pas plus d'un hétéroatome qui est N, O ou S dans le cycle;
et chaque $R_{10}$ indépendamment de l'autre est H ou un alkyle en $C_{1-4}$;

$R_6$ est H ou un alkyle en $C_{1-4}$;

$R_2$ est un atome de fluor ou de brome; et

$R_9$ est un atome d'hydrogène ou un groupe hydroxyle; et les composés qui sont les sels ou les sels d'addition d'acides convenant en pharmacie de ceux-ci.

2. Un composé comme revendiqué dans la revendication 1 dans lequel $R_7$ est $X-R_8$ et X est S.

3. Un composé comme revendiqué dans la revendication 1 dans lequel $R_7$ est $X-R_8$ et X est O.

4. Un composé comme revendiqué dans la revendication 1 dans lequel $R_7$ est $X-R_8$ et X est NH.

5. Les composés suivants revendiqués dans la revendication 1:
acide 4-(3-(4-acétyl-3-hydroxy-2-propylphénoxy)-2-hydroxypropoxy)-3-fluorobenzène-acétique;
acide 4-(3-(4-acétyl-3-hydroxy-2-propylphénoxy)-2-hydroxypropoxy)-3-bromobenzène-acétique;
acide 4-(3-(4-acétyl-3-hydroxy-2-propylphénoxy)-propoxy)-3-fluorobenzène-acétique;
acide 4-(3-(4-acétyl-3-hydroxy-2-propylphénoxy)-propoxy)-3-bromobenzène-acétique;
ester éthylique de l'acide 4-(3-(4-acétyl-3-hydroxy-2-propylphénoxy-2-hydroxypropoxy)-3-fluorobenzène-acétique;
4-(3-(4-acétyl-3-hydroxy-2-propylphénoxy-2-hydroxypropoxy)-3-fluorobenzène-éthanol;
4-(3-(4-acétyl-3-hydroxy-2-propylphénoxy-2-hydroxypropoxy)-3-fluorobenzène-acétaldéhyde;
4-(3-(4-acétyl-3-hydroxy-2-propylphénoxy-2-hydroxypropoxy)-3-fluorobenzèneméthyltétrazole;
N-méthylsulfonyl-4-(3-(4-acétyl-3-hydroxy-2-propylphénoxy-2-hydroxypropoxy)-3-fluorobenzène-éthyl-amine;
4-(3-(4-acétyl-3-hydroxy-2-propylphénoxy-2-hydroxypropoxy)-3-fluorobenzylnitrile.

6. Un procédé de préparation d'un composé répondant à la formule I indiquée dans la revendication 1 dans laquelle $R_9$ est un hydrogène, qui comprend la réaction d'un composé de formule II

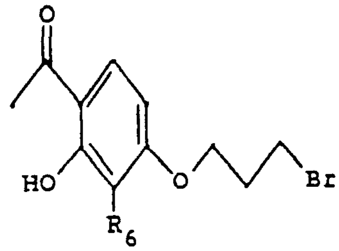

II

avec un composé de formule IV

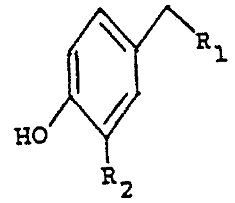

IV

où $R_1$, $R_2$ et $R_6$ sont comme défini dans la revendication 1.

7. Un procédé de préparation d'un composé répondant à la formule I indiquée dans la revendication 1 dans laquelle $R_9$ est un hydroxy, qui comprend la réaction d'un composé de formule III

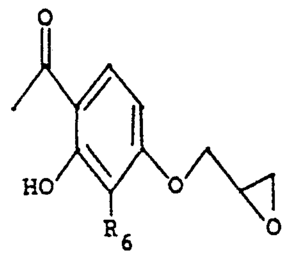

III

avec un composé de formule IV

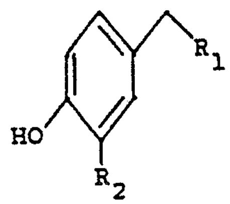

IV

où $R_1$, $R_2$ et $R_6$ sont comme défini dans la revendication 1.

8. Un procédé de préparation d'un composé répondant à la formule I indiquée dans la revendication 1 dans laquelle $R_9$ est un hydrogène, qui comprend la réaction d'un composé de formule V

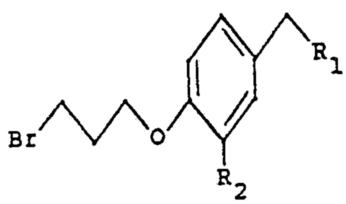

V

avec un composé de formule VII

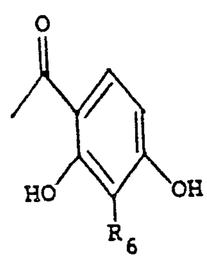

VII

où $R_1$, $R_2$ et $R_6$ sont comme défini dans la revendication 1.

9. Un procédé de préparation d'un composé répondant à la formule I indiquée dans la revendication 1 dans laquelle $R_9$ est un hydroxy, qui comprend la réaction d'un composé de formule VI

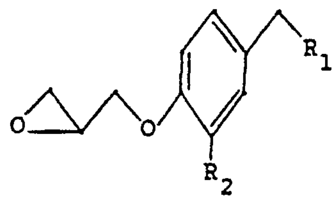

VI

avec un composé de formule VII

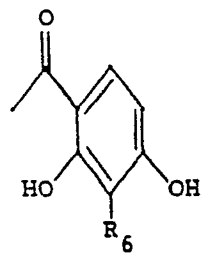

VII

où $R_1$, $R_2$ et $R_6$ sont comme défini dans la revendication 1.

10. Une composition contenant un composé comme revendique dans la revendication 1 et un support convenant en pharmacie.

11. Une composition pharmaceutique comprenant un composé comme revendiqué dans l'une quelconque des revendications 1 à 5 et de plus un second ingrédient actif qui est un médicament anti-inflammatoire non stéroïdien, un agent analgésique périphérique, un inhibiteur de la cyclooxygénase, un antagoniste du leucotriène, un inhibigeur du leucotriène, un antagoniste des récepteurs $H_2$, un agent antihistaminique, un antagoniste des prostaglandines ou un antagoniste du thromboxane.

12. Une composition comme revendiqué dans la revendication 11 dans laquelle le rapport pondéral du composé de l'une des revendications 1 à 5 au second ingrédient actif est compris dans la gamme de 200/1 à 1/200.

13. Une composition comme revendiqué dans la revendication 11 ou 12 dans laquelle le second ingrédient actif est un médicament anti-inflammatoire non stéroïdien.

14. Une composition comme revendiqué dans la revendication 13 dans laquelle le médicament anti-inflammatoire non stéroïdien est l'indométhacine.

15. Une composition comme revendiqué dans l'une quelconque des revendications 10 à 14 pour l'emploi comme antagoniste de la synthèse ou du mode d'action du leucotriène chez les humains.

16. Une composition comme revendiqué dans l'une quelconque des revendications 10 à 14 pour l'emploi dans le traitement de l'asthme, des troubles allergiques et de l'inflammation chez les humains.